# EUROPEAN PATENT SPECIFICATION

(11) **EP 3 702 784 B1**
(45) Date of publication and mention of the grant of the patent: **04.01.2023**
(21) Application number: 18871731.8
(22) Date of filing: 24.10.2018
(51) Int. Cl.: G01N 33/574

(54) **METHOD FOR DIAGNOSING CANCER FROM BLOOD WITH BAG2**
VERFAHREN ZUR DIAGNOSE VON KREBS AUS BLUT MIT BAG2
MÉTHODE DE DIAGNOSTIC DU CANCER À PARTIR DU SANG AVEC BAG2

(30) Priority: 24.10.2017 KR 20170138455
(43) Date of publication of application: 02.09.2020
(73) Proprietor: Medpacto Inc., Seoul 06668 (KR)
(72) Inventor: KIM, Seong Jin, Suwon-si Gyeonggi-do 16229 (KR); YANG, Kyung Min, Incheon 21995 (KR)
(74) Representative: Russell, Tim
(86) International application number: PCT/KR2018/012591
(87) International publication number: WO 2019/083262

(56) References cited:
- KR-A- 20100 112 192
- US-A1- 2007 105 133
- US-A1- 2010 255 470
- Yue Xuetian ET AL: "BAG2 promotes tumorigenesis through enhancing mutant p53 protein levels and function", eLife, 13 August 2015 (2015-08-13), XP55796117, England DOI: 10.7554/eLife.08401 Retrieved from the Internet: URL:https://www.ncbi.nlm.nih.gov/pmc/artic les/PMC4561369/pdf/elife08401.pdf [retrieved on 2021-04-16]
- QIN LIXIA ET AL: "BAG2 structure, function and involvement in disease", CELLULAR & MOLECULAR BIOLOGY LETTERS, vol. 21, no. 1, 20 September 2016 (2016-09-20), XP55796025, PL ISSN: 1425-8153, DOI: 10.1186/s11658-016-0020-2 Retrieved from the Internet: URL:http://link.springer.com/content/pdf/1 0.1186/s11658-016-0020-2.pdf>
- DATABASE PROTEIN 23 March 2015 (2015-03-23), VENTER J:C: et al.: "BCL2-associated athanogene 2 [Homo sapiens)", XP055595392, retrieved from NCBI Database accession no. EAX04475
- DATABASE PROTEIN 7 April 1994 (1994-04-07), CAO L. et al.: "cathepsin B [Homo sapiens", XP055595393, retrieved from NCBI Database accession no. AAC37547
- YANG, KYUNG-MIN: "Co-chaperone BAG2 determines the pro-oncogenic role of cathepsin B in triple-negative breast cancer cells", Cell Reports, vol. 21, no. 1010, 5 December 2017 (2017-12-05), pages 2952-2964, XP055595394, ISSN: 2211-1247, DOI: 10.1016/j.celrep.2017.11.026
- Exhibit A
- HUNG CHIUNG-HUI ET AL: "Prediction of non-classical secreted proteins using informative physicochemical properties", INTERDISCIPLINARY SCIENCES: COMPUTATIONAL LIFE SCIENCES, vol. 2, no. 3, 1 September 2010 (2010-09-01), pages 263-270, XP55896258, CA ISSN: 1913-2751, DOI: 10.1007/s12539-010-0023-z Retrieved from the Internet: URL:https://link.springer.com/content/pdf/ 10.1007/s12539-010-0023-z.pdf>
- NIELSEN HENRIK ET AL: "Predicting eukaryotic protein secretion without signals", BIOCHIMICA ET BIOPHYSICA ACTA (BBA) - PROTEINS & PROTEOMICS, ELSEVIER, NETHERLANDS, vol. 1867, no. 12, 4 December 2018 (2018-12-04), XP085882862, ISSN: 1570-9639, DOI: 10.1016/J.BBAPAP.2018.11.011 [retrieved on 2018-12-04]

## Description

### TECHNICAL FIELD

The present disclosure relates to a method of diagnosing cancer in serum, plasma or a combination thereof using soluble BAG2.

### BACKGROUND ART

Co-chaperone Bcl-2-associated athanogene (BAG) protein family mediates diverse physiological processes, including intracellular protein folding, stress response, neuronal differentiation, cell death, cell proliferation, etc., and functionally binds to a variety of binding partners (Takayama and Reed., 2001; Doong et al., 2002, Qin et al., 2016). Among them, BAG2 which is a member of BAG domain family with anti-apoptotic activity has been known as a negative regulator of C terminus of Hsc70-interacting protein (CHIP) which is a chaperone-associated ubiquitin ligase (Arndt et al., 2005; Dai et al., 2005). The main effect of BAG2 on the control of protein through inhibition of CHIP activity is linked to neurodegenerative disorders and autosomal-recessive disorders through protein stabilization of chaperones, such as PINK1 and CFTR (Qu et al., 2015; Arndt et al., 2005). It has been reported that BAG2 expression increases proteasome-inhibitor-induced apoptosis, and BAG2 knockdown partially inhibits cell death in thyroid carcinoma cells exposed to a proteasome inhibitor MG132, suggesting that BAG2 may have pro-apoptotic activity (Wang et al., 2008). Meanwhile, it has been suggested that in various mutant K-Ras-driven tumors, overexpression of BAG2 is able to promote tumor development by promoting the stabilization of STK33 protein which is a potent tumor gene (Azoitei et al., 2012). A role of BAG2 in mutant p53 accumulation has been suggested (Xuetian Yue et al., 2015). However, despite these findings, the role of BAG2 in cancer progression and metastasis, particularly, in breast cancer has not been clearly revealed.

Cathepsin B (CTSB), which is a lysosomal cysteine protease possessing both endo- and exo-peptidase activity, is considered to participate in protein turnover (Mort and Buttle, 1997). CTSB is synthesized as an inactive/immature pro-form enzyme (41/43 kDa), which is converted to an active single-chain form (31 kDa) or double-chain form (heavy chain, 25/26 kDa; light chain, 5 kDa) through proteolytic processing of a 62-amino acid propeptide of the N-terminus. Aberrant expression/activity of CTSB has often been linked to malignant tumors (Joyce et al., 2004; Withans, 2012). However, it has also been recognized that mature CTSB is normally localized in lysosomes, where it functions in protein turnover as an initiator protease during lysosome-mediated autophagy/apoptosis, indicating a pro-apoptotic role for cytosolic CTSB (Stoka et al., 2001; Foghsgaard et al., 2001; Bhoopathi et al., 2010). Meanwhile, regulators that control the dual functions of CTSB in cancer progression have not been clearly revealed.

Furthermore, cellular location and distribution of BAG2 and cathepsin B, and their change of localization according to cell functions have not been studied in detail.

### DESCRIPTION OF EMBODIMENTS

### TECHNICAL PROBLEM

The claimed invention generally provides a method as claimed in claim 1.

### SOLUTION TO PROBLEM

Hereinafter, the present disclosure will be described in more detail.

Unless defined otherwise, all technical terms used herein have the same meanings as those generally understood by one of ordinary skill in the art to which the present disclosure belongs.

An aspect provides a method of diagnosing cancer, the method including bringing a sample isolated from an individual into contact with an antibody, a polypeptide or a combination thereof, which specifically binds to a BAG2 polypeptide or a fragment thereof; measuring the presence or level of BAG2 in the sample from a complex formed by coming into contact with the antibody, the polypeptide, or the combination thereof, which specifically binds to the BAG2 polypeptide or the fragment thereof; and comparing the presence or level of BAG2 measured from the sample with a BAG2 level measured from a control group. BAG2 comprises the amino acid sequence of SEQ ID NO:1 or 2, or a variant having 80% or more sequence identity to SEQ ID NO:1 or 2. The sample is serum, plasma, or a combination thereof. The BAG2 is soluble in serum, plasma, or a combination thereof.

When BAG2 is present in a sample isolated from an individual, an antibody, a polypeptide, or a combination thereof specifically binding to a BAG2 polypeptide or a fragment thereof may bind to BAG2 in the sample. The antibody or the polypeptide may be labeled with, for example, a fluorophore, a chromophore, or an enzyme capable of converting a substrate into a chromophore to visualize the presence of BAG2 in the sample.

Through the binding reaction, BAG2 in the sample forms a complex with the antibody, the polypeptide, or the combination thereof which specifically binds to the BAG2 polypeptide or the fragment thereof. From the complex, the presence of BAG2, or as needed, the level thereof, may be measured using a method commonly known to those skilled in the art. In a specific embodiment, the measuring of the complex may be performed by Western blotting, enzyme linked immunosorbent assay (ELISA), radioimmunoassay (RIA), radioimmunodiffusion, Ouchterlony immunodiffusion, rocket immunoelectrophoresis, immunohistostaining, an immunoprecipitation assay, a complement fixation assay, fluorescence activated cell sorting (FACS), a protein chip, or a combination thereof.

Whether cancer is present in the individual may be diagnosed by comparing the presence or level of BAG2 measured from the sample with a BAG2 level measured from a control group. The control group may be a sample collected from a healthy individual, or as needed, a sample collected from a mean of patients with various types of breast cancers, a patient with non-metastatic breast cancer, or a patient with non-triple negative breast cancer (TNBC). Therefore, the BAG2 level measured from the control group may refer to a mean value of BAG2 levels in the sample collected from the healthy individual, or as needed, the sample collected from a mean of patients with various types of breast cancers, the patient with non-metastatic breast cancer, or the patient with non-TNBC. The sample used as the control group may be of the same kind collected from the same anatomical site as the sample for diagnosis. For example, when the sample is from blood collected from a median cubital vein of an individual, the control group may also be from blood collected from a median cubital vein of the control group. The healthy individual may be an individual not suffering from any acute or chronic disease, and may be an individual at least not suffering from cancer, and an individual not suffering from breast cancer.

The BAG2 level in the sample collected from the healthy individual may mean a substantial absence of BAG2. Therefore, when a value obtained from the healthy individual is determined as the control group, and the presence of BAG2 is detected or the level thereof is remarkably high in an individual as a subject of diagnosis, the individual may be suspected of having cancer. In contrast, as shown in embodiments of the present disclosure, the presence of BAG2 is likely to be detected and the mean value thereof is significantly high in blood of patients with metastatic breast cancer, in particular, patients with TNBC, among patients with breast cancer. Therefore, when the sample collected from a mean of patients with various types of breast cancers, the patient with non-metastatic breast cancer, or the patient with non-TNBC is determined as the control group, and a BAG2 level in an individual as a subject of diagnosis is higher or remarkably higher than that of the control group, the individual may be suspected of having metastatic breast cancer or TNBC.

In the present disclosure, the 'polypeptide' and 'protein' may be used interchangeably.

The sample may be isolated from an individual as a subject of diagnosis, and may be serum, plasma, or a combination thereof.

In a specific embodiment, the sample may be serum, plasma, or a combination thereof. The present inventors demonstrated that BAG2 may be secreted from cells and BAG2 is actually detected in the blood of breast cancer patients. In a specific embodiment, the BAG2 may be soluble in blood, serum, plasma, or a combination thereof.

In a specific embodiment, the diagnostic method of the present disclosure may further include bringing the sample isolated from the individual into contact with an antibody, a polypeptide or a combination thereof, which specifically binds to a cathepsin B polypeptide or a fragment thereof; measuring the presence or level of cathepsin B from a complex formed by coming into contact with the antibody, the polypeptide, or the combination thereof, which specifically binds to the cathepsin B polypeptide or the fragment thereof; and comparing the presence or level of cathepsin B measured from the sample with a cathepsin B level measured from a control group.

The present inventors demonstrated that BAG2 prevents the conversion of cathepsin B to a mature form, and eventually suppressing cancer cell apoptosis, and consistent with this, they observed that both BAG2 and an immature form of cathepsin B are secreted from cells. Therefore, the diagnostic method of the present disclosure may detect BAG2 and cathepsin B at the same time, thereby further increasing diagnostic accuracy.

Cathepsin B may be divided into a pro-cathepsin B and a single-chain or double-chain cathepsin B as a mature form, and the present inventors demonstrated that an immature form of cathepsin B is secreted from cells by BAG2. Therefore, in a specific embodiment, in the diagnostic method of the present disclosure, the cathepsin B may be pro-cathepsin B. The pro-cathepsin B may be used in the same meaning as 'immature cathepsin B' or 'immature CTSB'.

In a specific embodiment, the measuring of the complex formed by bringing cathepsin B in the sample into contact with the antibody, the polypeptide, or the combination thereof, which specifically binds to the cathepsin B polypeptide or the fragment thereof may be performed by Western blotting, ELISA, RIA, radioimmunodiffusion, Ouchterlony immunodiffusion, rocket immunoelectrophoresis, immunohistostaining, an immunoprecipitation assay, a complement fixation assay, FACS, a protein chip, or a combination thereof.

The term "antibody" refers to a specific immunoglobulin directed against an antigenic site. The antibody refers to a polypeptide or a combination of polypeptides, which specifically binds to BAG2 and pro-cathepsin B, respectively, and a protein encoded by BAG2 or pro-cathepsin B gene may be obtained by cloning the BAG2 or pro-cathepsin B gene into an expression vector, and an antibody specific to each protein may be prepared from the produced BAG2 or pro-cathepsin B protein using a common method in the art. The antibody includes a polyclonal antibody, a monoclonal antibody, or a recombinant antibody (e.g., ScFv fragment, diabody, single-chain antibody, etc.). All immunoglobulin antibodies are included. The antibody includes not only a complete form having two full-length light chains and two full-length heavy chains, but also functional fragments of antibody molecules having an antigen-binding function due to having a specific antigen-binding site (binding domain) directed against an antigenic site, although the antibody does not have a complete intact antibody structure having two light chains and two heavy chains.

The BAG2 and pro-cathepsin B polypeptide may be derived from human (*Homo sapiens*) or mouse (*Mus musculus*), respectively, or may be derived from other mammals such as monkey, cow, horse, etc. An amino acid sequence of BAG2 may include SEQ ID NO: 1 or 2 (GenBank Accession No. NP_004273.1 and NP_663367.1, respectively), and an amino acid sequence of pro-cathepsin B may include SEQ ID NOS: 3 to 8 (GenBank Accession No. NP_001899.1, NP_680090.1, NP_680091.1, NP_680092.1, NP_680093.1, or NP_031824.1, respectively). Although amino acid sequences are partially inconsistent with the amino acid sequences of SEQ ID NOS: 1 to 8, the amino acid sequences may be regarded as the BAG2 or pro-cathepsin B polypeptide as long as the amino acid sequences have activity biologically equivalent thereto. The BAG2 or pro-cathepsin B polypeptide may include an amino acid sequence having 80% or more, 90% or more, 95% or more, 99% or more, or 100% sequence identity to any one of SEQ ID NO: 1 or 2, and SEQ ID NO: 3 or 8, respectively. Further, the BAG2 or pro-cathepsin B polypeptide may be a polypeptide having a sequence including one or more amino acid residues, two or more amino acid residues, three or more amino acid residues, four or more amino acid residues, five or more amino acid residues, six or more amino acid residues, or seven or more amino acid residues different from any one sequence of SEQ ID NO: 1 or 2, and SEQ ID NO: 3 or 8, respectively.

The individual as a subject of the diagnostic method may be a mammal, for example, a human, a mouse, a rat, a cow, a horse, a pig, a dog, a sheep, a goat, or a cat, and specifically, a human.

In a specific embodiment, cancer which may be diagnosed by the diagnostic method of the preset disclosure may be selected from breast cancer, colorectal cancer, lung cancer, sarcoma, melanoma, head and neck cancer, cervical cancer, uterine cancer, liver cancer, renal cancer, pancreatic cancer, and neuroblastoma.

The present inventors demonstrated that BAG2 is overexpressed in breast cancer patients, and in particular, breast cancer patients with high BAG2 levels are at high risk of developing metastatic breast cancer. Therefore, in a specific embodiment, breast cancer which may be diagnosed by the diagnostic method of the present disclosure may be metastatic breast cancer.

Among the molecular subtypes of breast cancer, triple-negative breast cancer (TNBC) is an extremely aggressive subtype that is correlated with a poor prognosis and high mortality rates despite systemic therapy. TNBC is also a heterogeneous type compared to either a luminal type or a HER2-enriched type (Foulkes et al., 2010; Dent et al., 2007; Masuda et al., 2013). Most breast cancer targeted therapies have shown positive outcomes for hormone receptor- and HER2-positive breast cancers, whereas TNBC patients have limited options for effective treatment (e.g., poly ADP-ribose polymerase (PARP), epidermal growth factor receptor (EGFR), and Src tyrosine kinase, etc.) due to the absence of three target receptors (ER, PR, and HER2) or other well-defined molecular targets. Therefore, it is necessary to quickly and accurately distinguish TNBC patients from patients with various types of breast cancers, in order to prevent unnecessary therapeutic approaches and to select effective treatments for TNBC patients.

The present inventors demonstrated that BAG2 is overexpressed in breast cancer patients, and in particular, breast cancer patients with high BAG2 levels are at high risk of developing TNBC. Therefore, in a specific embodiment, breast cancer which may be diagnosed by the diagnostic method of the present disclosure may be triple-negative breast cancer (or TNBC).

Another aspect, which is not encompassed by the claims, provides a kit for diagnosing cancer, the kit including an antibody, a polypeptide, or a combination thereof which specifically binds to the BAG2 polypeptide or the fragment thereof. The kit is to detect BAG2 present in blood, serum, plasma, or a combination thereof, and a sample applied to the kit may be blood, serum, plasma, or a combination thereof, which is isolated from an individual.

The kit may further include an antibody, a polypeptide, or a combination thereof which specifically binds to the cathepsin B polypeptide or the fragment thereof.

The kit may further include one or more other constitutional compositions, solutions, or apparatuses that are suitable for an analysis method (e.g., Western blotting, ELISA, RIA, radioimmunodiffusion, Ouchterlony immunodiffusion, rocket immunoelectrophoresis, immunohistostaining, an immunoprecipitation assay, a complement fixation assay, FACS, a protein chip, or a combination thereof) employed in the kit. For example, the kit may further include a substrate, a suitable buffer, a secondary antibody labeled with a chromogenic enzyme or a fluorophore, and a chromogenic substrate to detect an immune complex of BAG2 or cathepsin B in the sample and an antibody specific thereto. The substrate may be a nitrocellulose membrane, a 96-well plate synthesized from a polyvinyl resin, a 96-well plate synthesized from a polystyrene resin, a glass slide, etc., the chromogenic enzyme may be peroxidase or alkaline phosphatase, the fluorophore may be FITC, RITC, etc., and the chromogenic substrate may be 2,2'-azino-bis(3-ethylbenzothiazoline-6-sulfonic acid) (ABTS), o-phenylenediamine (OPD), tetramethyl benzidine (TMB), etc.

### ADVANTAGEOUS EFFECTS OF DISCLOSURE

According to a method of diagnosing cancer by measuring the presence or level of a BAG2 polypeptide in a sample from blood according to an aspect, it is possible to more easily and accurately diagnose cancer than existing methods in which tissues are collected for cancer diagnosis.

According to a cancer diagnostic kit for measuring the presence or level of a BAG2 polypeptide in a sample from blood, the method of diagnosing cancer may be easily utilized by various diagnostic institutions for cancer diagnosis.

### BRIEF DESCRIPTION OF DRAWINGS

FIG. 1 shows a volcano plot analyzing differential expression levels of BAG family in luminal type and TNBC cell lines using RNA sequencing;
FIG. 2 shows BAG2 mRNA expression levels in luminal type and TNBC cell lines, which were analyzed by the volcano plot;
FIG. 3 shows scatter dot plots of BAG2 expression levels which were analyzed in 52 breast cancer cell lines;
FIG. 4 shows microarray (4A) and RNA sequencing results (4B) of BAG2 expression levels in normal and respective breast cancer cell types;
FIG. 5 shows quantitative RT-PCR results of analyzing BAG2 expression levels in luminal type and TNBC tissues;
FIG. 6 shows immunoblot (6A) and CTSB activity (6B) to analyze effects of BAG2 on CTSB form and activity;
FIG. 7 shows immunoblotting results of detecting BAG2 and CTSB in TNBC-cultured conditioned media;
FIG. 8 shows a graph of pro-cathepsin B levels detected in TNBC-cultured conditioned media;
FIG. 9 shows immunoblotting results of detecting BAG2 and CTSB in conditioned media in which a BAG2-overexpressed cell line was cultured; and
FIG. 10 shows results of detecting the presence of BAG2 in sera of healthy persons and breast cancer patients.

### MODE OF DISCLOSURE

### 1. Example 1: Examination of BAG2 Expression in Various Breast Cancer Cell Lines

### 1-1. Transcriptome analysis of breast cancer cell lines

To identify novel molecular targets associated with TNBC progression, the present inventors initially performed transcriptome analysis using RNA sequencing in breast cancer cell lines. First, RNAs were extracted from various breast cancer cell lines using a method well known in the art, respectively, and genome sequencing was performed by a company TheragenEtex. Thereafter, the breast cancer cell lines were classified as either the luminal type (MCF-7, T47D, ZR-75B) or TNBC (MDA-MB-231, Hs578T), and BAG2 expression levels were compared.

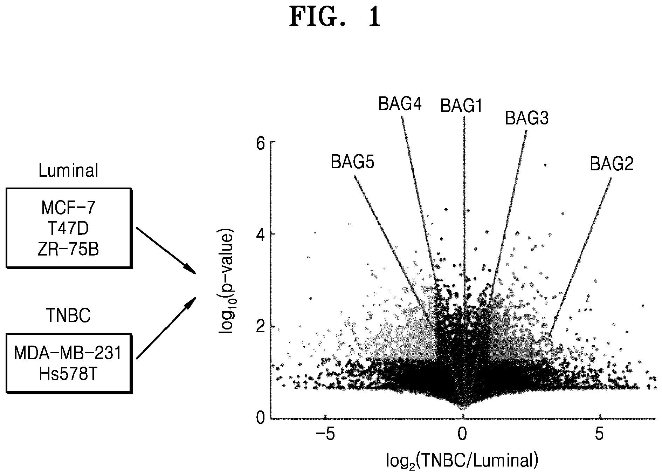

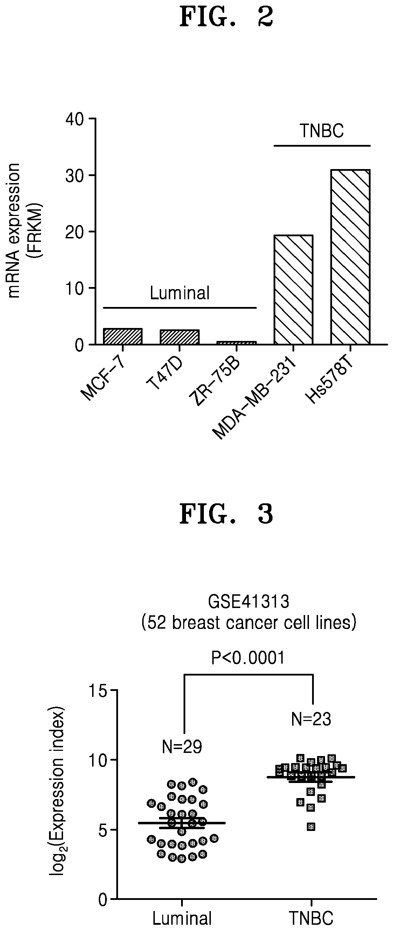

As a result, as shown in FIGS. 1 and 2, BAG2 was specifically overexpressed in TNBC cell lines, as compared with luminal type cell lines.

### 1-2. Examination of BAG2 expression patterns in 52 breast cancer cell lines

Extended from Example 1-1, BAG2 expression patterns in more various breast cancer cells were examined. Information of breast cancer cells was obtained from public microarray datasets (GSE41313, Riaz et al., 2013). BAG2 expression patterns were examined in 52 breast cancer cell lines published by Riaz et al., and as a result, BAG2 expression was significantly higher in
TNBC cells than in luminal types (P<0.0001), as shown in FIG. 3.

### 1-3. Examination of clinical BAG2 expression patterns

For more accurate examination of BAG2 expression patterns which were examined in the cell lines, the present inventors analyzed BAG2 expression patterns in different subtypes of breast cancer using RNA sequencing datasets of breast cancer patients obtained from The Cancer Genome Atlas (TCGA) and microarrays.

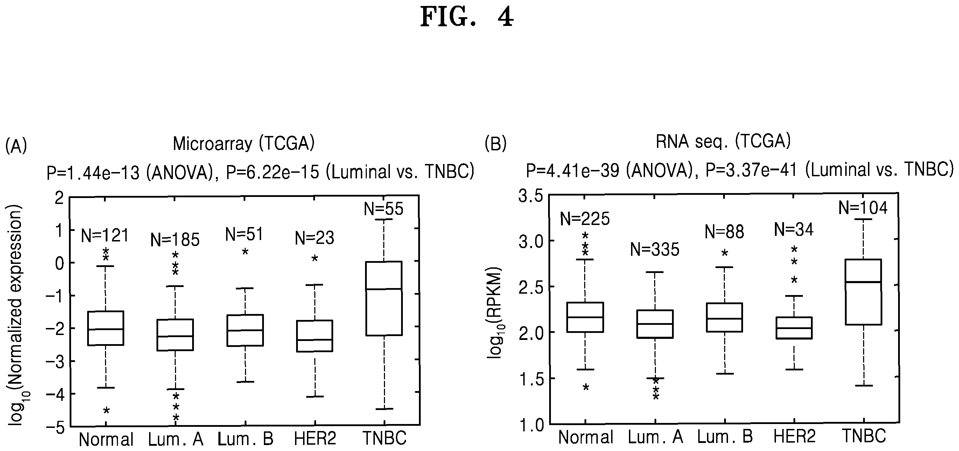

As a result, as shown in FIGS. 4A and 4B, a remarkably high level of BAG2 expression was observed in TNBC patients, as compared with normal, luminal A (Lum A), luminal B (Lum B), and HER2-overexpressing (HER2) patients.

### 1-4. Experimental examination of BAG2 expression patterns

To further experimentally confirm the patterns analyzed using the public databases, quantitative RT-PCR was performed.

In detail, 12 luminal and 6 TNBC breast cancers were obtained from Gangnam Severance Hospital after approval by the institutional review board (IRB approval number 3-2013-0268). Thereafter, total RNAs were isolated from cells according to the manufacturer's instruction using TRIzol reagent (Invitrogen). Then, quantitative RT-PCR was performed using 18s rRNA as a housekeeping gene, primers for BAG2 detection, power SYBR Green PCR Master Mix and Viia 7 real-time PCR device (Applied Biosystems).

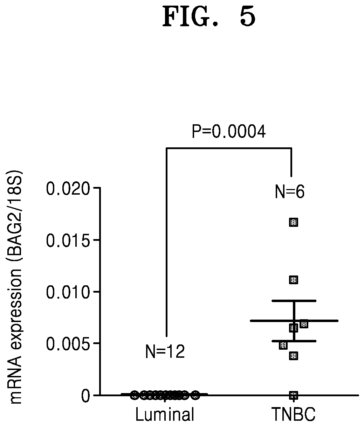

As a result, as shown in FIG. 5, BAG2 overexpression was observed in TNBC, as compared with luminal.

### 2. Example 2: Examination of Interaction between BAG2 and Cathepsin B

To examine whether BAG2 acts as an upstream regulator of cathepsin B, interaction between BAG2 and cathepsin B and change of cathepsin B activity by BAG2 were examined.

### 2-1. Examination of interaction between BAG2 and cathepsin B at protein level

To prepare BAG2-depleted cell line, BAG2-specific shRNA was first purchased from Mission-shRNA (Sigma). To prepare BAG2-specific shRNA lentivirus, 293T cells were transfected with pLKO-BAG2 or scrambled control pLKO-pGL2, together with packaging plasmids encoding Δ8.9 and VSV-G (target sequence of used BAG2 shRNA (5'→ 3'): GTACTAGGATCTAGCATATTT). Viral supernatants were collected 36 hr and 48 hr post-transfection, and filtered through a 0.45-µm filter. MDA-MB-231 (ATCC) was seeded at 1 × 10⁵ cells/well into 6-well plates, and infected with viral particles and polybrene (8 µg/ml). After the infection, the virus-containing medium was replaced with a normal medium, and then the cells were selected by puromycin (2 µg/ml). The prepared BAG2-depleted cell line was represented by BAG2 shRNA.

To overexpress BAG2, MDA-MB-231 (ATCC) was also transfected with a Myc-tag BAG2 expression vector according to the manufacturer's instruction using Lipofectamine 2000 (Invitrogen). BAG2-overexpressed cells were represented by BAG2 OV, and BAG2-rescued cells prepared by transfecting BAG2 shRNA with BAG2 were represented by BAG2 rescue.

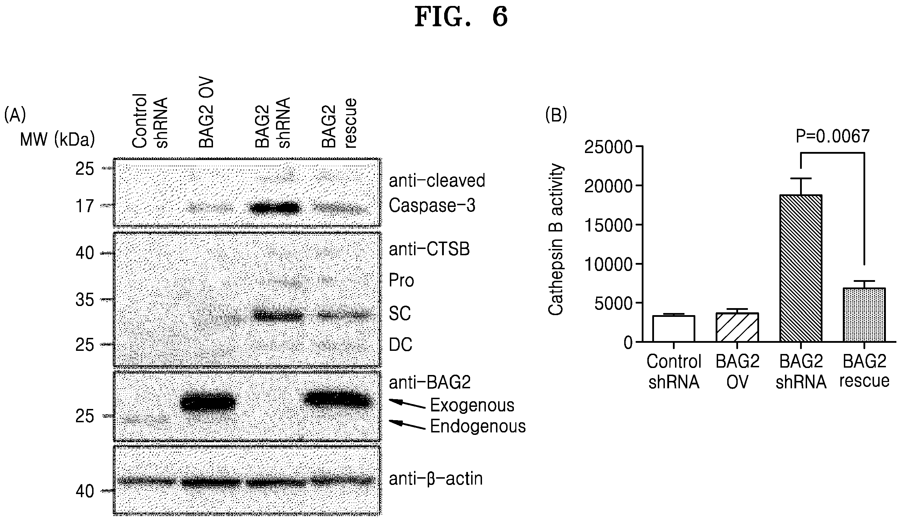

The respective cells were washed with PBS, and lysed, and then separated by SDS-polyacrylamide gel electrophoresis, transferred to PVDF membranes (Millipore), and respective proteins were detected by immunoblotting using antibodies shown in FIG. 6. In particular, because CTSB antibody recognizes both the pro-form and the mature forms of CTSB (single chain (SC) and double chain (DC)), they may be distinguished by their molecular weights.

As a result, as shown in FIG. 6A, cleaved caspase-3 was expressed in BAG2-depleted cells, indicating an increase in active caspase-3, which was then decreased by BAG2 rescue. Further, single chain (SC) cathepsin B was also increased in BAG2-depleted cells, but decreased by BAG2 rescue, like caspase-3. Therefore, it is suggested that BAG2 is involved in inhibiting conversion of pro-cathepsin B into single chain (SC) cathepsin B as a mature form.

### 2-2. Examination of effect of BAG2 on CTSB activity

To further verify the effect of BAG2 on CTSB control, changes in CTSB activity depending on the presence of BAG2 were observed.

In detail, BAG2-depleted MCF10CA1a cells were prepared as in Example 3-1, and transfected with a BAG2 expression vector to overexpress BAG2, or BAG2 expression was rescued in BAG2-depleted cells. A substrate of mature CTSB is a synthetic peptide containing Arg-Arg, and the prepared cell sample was incubated in the presence of a fluorogenic cathepsin B substrate (200 µM Ac-RR-AFC; Abcam) for 1 hr at 27°C according to the manufacturer's instructions. Fluorescence measurements were conducted on a multiplate reader Wallac 1440 Victor2 (PerkinElmer) and relative fluorescent units were measured at 460 nm using an excitation wavelength of 355 nm.

As a result, as shown in FIG. 6B, when BAG2 was re-introduced to BAG2-depleted MCF10CA1a cells, increased CTSB activity was observed by BAG2 deficiency, suggesting that BAG2 inhibits conversion of CTSB to the mature form, leading to reduction in CTSB activity. Therefore, it is suggested that BAG2 interacts with CTSB to inhibit conversion of CTSB to the mature form, and thus BAG2 is involved in inhibiting caspase-3-mediated apoptosis.

### Example 3. Examination of Presence of BAG2 and CTSB in Blood

### 3-1. Examination of BAG2 and CTSB secretion by TNBC cells

It was examined whether BAG2 and CTSB were not only present inside TNBC cells but also secreted from the cells.

In detail, Hs578T, MDA-MB-231, and MCF10ACa1 as TNBC cells were used to prepare BAG2-depleted cells as in Example 2-1, and then respective cells were cultured in conditioned DMEM (Cat. LM001-05, WELGENE), and supernatants were collected while taking care not to collect the cells, followed by immunoblotting as in Example 2-1. To visualize the total amount of proteins, a portion thereof was stained with coomassie blue (ELT006, ELBIO) according to the manufacturer's instruction, respectively.

Further, a portion of each sample obtained from the medium was analyzed using a pro-cathepsin B quantikine ELISA kit (R&D system) according to the manufacturer's instruction, and the presence of BAG2 and CTSB was further examined in the culture medium of MCF10AT1 which is a BAG2-overexpressed cell line.

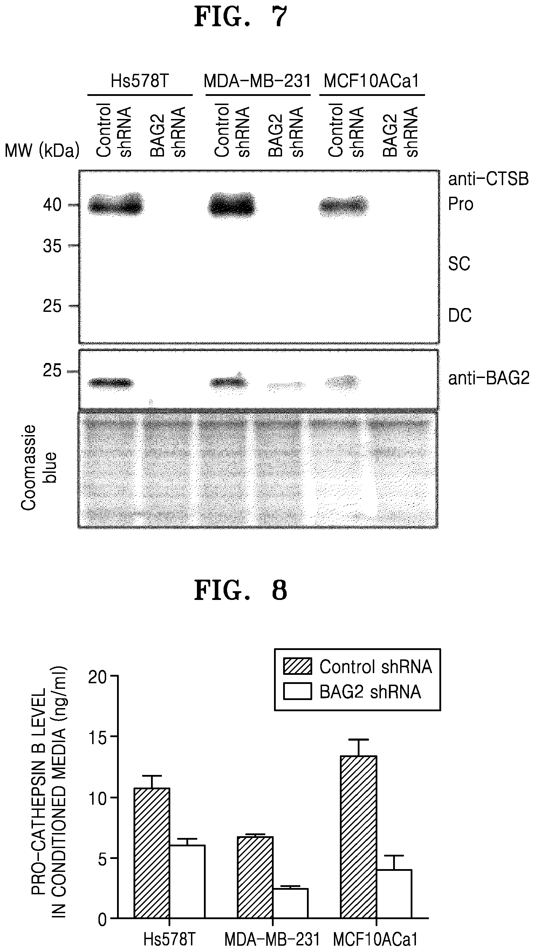

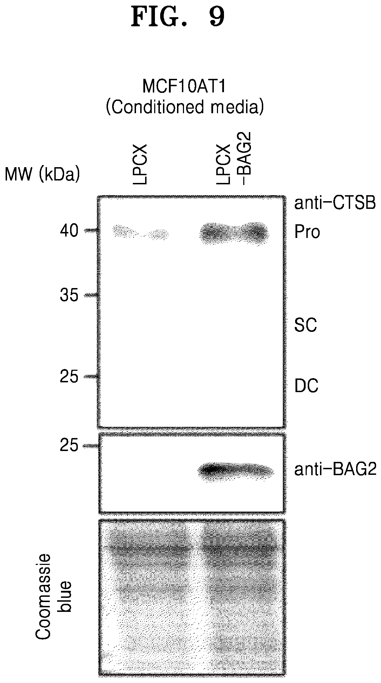

As a result, as shown in FIGS. 7 to 9, both BAG2 and CTSB were observed in the culture media of BAG2-expressed TNBC cells. In particular, CTSB as a pro-CTSB showed the same pattern as BAG2 expression.

### 3-2. Examination of Blood BAG2 and CTSB secretion

Extracellular secretion of BAG2 was confirmed, and thus it was further examined whether BAG2 was also secreted into blood, and thus detected in the blood.

In detail, sera were collected from healthy volunteers (N=17) and malignant breast cancer patients (N=76). The obtained sera were analyzed using a human BAG2 ELISA kit (MyBiosource) according to the manufacturer's instruction, respectively.

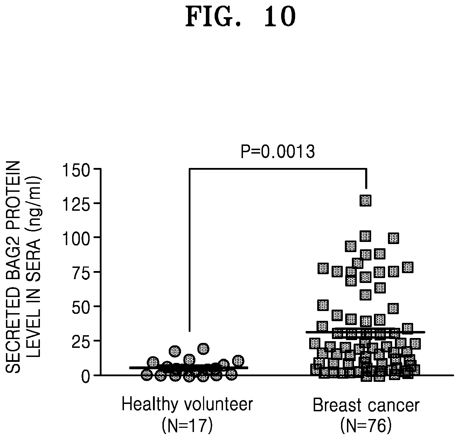

As a result, as shown in FIG. 10, significant levels of BAG2 were detected in the sera of breast cancer patients, as compared with those in healthy volunteers.

In the above embodiments, p values were calculated by unpaired two-tailed Student's t-test, and represented by means ± SD of three independent experiments.

Hereinabove, the present disclosure has been described with reference to exemplary embodiments thereof. Exemplary embodiments disclosed herein should be considered in an illustrative aspect rather than a restrictive aspect.

## Claims

1. A method of diagnosing cancer, the method comprising:
bringing a sample isolated from an individual into contact with an antibody, a polypeptide or a combination thereof, which specifically binds to a BAG2 polypeptide or a fragment thereof;
measuring the presence or level of BAG2 in the sample from a complex formed by coming into contact with the antibody, the polypeptide, or the combination thereof, which specifically binds to the BAG2 polypeptide or the fragment thereof; wherein BAG2 comprises i) the amino acid sequence of SEQ ID NO:1 or 2, or ii) a variant having 80% or more sequence identity to SEQ ID NO:1 or 2, and
comparing the presence or level of BAG2 measured from the sample with a BAG2 level measured from a control group,
wherein the sample is serum, plasma, or a combination thereof, and
wherein the BAG2 is soluble in serum, plasma, or a combination thereof.

2. The method of diagnosing cancer of claim 1, wherein an amino acid sequence of the BAG2 polypeptide has an amino acid sequence of SEQ ID NO: 1 or 2.

3. The method of diagnosing cancer of claim 1, further comprising bringing the sample isolated from the individual into contact with an antibody, a polypeptide or a combination thereof, which specifically binds to a cathepsin B polypeptide or a fragment thereof;
measuring the presence or level of cathepsin B from a complex formed by coming into contact with the antibody, the polypeptide, or the combination thereof, which specifically binds to the cathepsin B polypeptide or the fragment thereof; and
comparing the presence or level of cathepsin B measured from the sample with a cathepsin B level measured from a control group.

4. The method of diagnosing cancer of claim 3, wherein the cathepsin B is a pro-cathepsin B.

5. The method of diagnosing cancer of claim 3, wherein an amino acid sequence of the cathepsin B polypeptide has any one amino acid sequence selected from SEQ ID NOS: 3 to 8.

6. The method of diagnosing cancer of claim 3, wherein the measuring of the presence or level of BAG2 or cathepsin B in the sample is performed by Western blotting, enzyme linked immunosorbent assay (ELISA), radioimmunoassay (RIA), radioimmunodiffusion, Ouchterlony immunodiffusion, rocket immunoelectrophoresis, immunohistostaining, an immunoprecipitation assay, a complement fixation assay, fluorescence activated cell sorting (FACS), a protein chip, or a combination thereof.

7. The method of diagnosing cancer of claim 1, wherein the cancer is selected from breast cancer, colorectal cancer, lung cancer, sarcoma, melanoma, head and neck cancer, cervical cancer, uterine cancer, liver cancer, renal cancer, pancreatic cancer, and neuroblastoma.

8. The method of diagnosing cancer of claim 7, wherein the breast cancer is metastatic breast cancer.

9. The method of diagnosing cancer of claim 7, wherein the breast cancer is triple-negative breast cancer.

## Patentansprüche

1. Verfahren zum Diagnostizieren von Krebs, wobei das Verfahren umfasst:
Inkontaktbringen einer von einem Individuum isolierten Probe mit einem Antikörper, einem Polypeptid oder einer Kombination davon, der/das spezifisch an ein BAG2-Polypeptid oder ein Fragment davon bindet;
Messen des Vorliegens oder Spiegels von BAG2 in der Probe aus einem Komplex, der durch Inkontaktkommen mit dem Antikörper, dem Polypeptid oder der Kombination davon gebildet wird und der spezifisch an das BAG2-Polypeptid oder das Fragment davon bindet; wobei BAG2 i) die Aminosäuresequenz von SEQ ID NO:1 oder 2 oder ii) eine Variante mit 80 % oder mehr Sequenzidentität zu SEQ ID NO:1 oder 2 umfasst, und
Vergleichen des gemessenen Vorliegens oder Spiegels von BAG2 aus der Probe, mit einem gemessenen BAG2-Spiegel aus einer Kontrollgruppe,
wobei die Probe Serum, Plasma oder eine Kombination davon ist, und
wobei das BAG2 in Serum, Plasma oder einer Kombination davon löslich ist.

2. Verfahren zum Diagnostizieren von Krebs nach Anspruch 1, wobei eine Aminosäuresequenz des BAG2-Polypeptids eine Aminosäuresequenz von SEQ ID NO: 1 oder 2 aufweist.

3. Verfahren zum Diagnostizieren von Krebs nach Anspruch 1, ferner umfassend Inkontaktbringen einer von dem Individuum isolierten Probe mit einem Antikörper, einem Polypeptid oder einer Kombination davon, der/das spezifisch an ein Cathepsin-B-Polypeptid oder ein Fragment davon bindet;
Messen des Vorliegens oder Spiegels von Cathepsin B aus einem Komplex, der durch Inkontaktkommen mit dem Antikörper, dem Polypeptid oder der Kombination davon gebildet wird und der spezifisch an das Cathepsin-B-Polypeptid oder das Fragment davon bindet; und Vergleichen des gemessenen Vorliegens oder Spiegels von Cathepsin B aus der Probe, mit einem gemessenen Cathepsin-B-Spiegel aus einer Kontrollgruppe.

4. Verfahren zum Diagnostizieren von Krebs nach Anspruch 3, wobei das Cathepsin B ein Pro-Cathepsin B ist.

5. Verfahren zum Diagnostizieren von Krebs nach Anspruch 3, wobei eine Aminosäuresequenz des Cathepsin-B-Polypeptids eine beliebige Aminosäuresequenz aufweist, ausgewählt aus SEQ ID NO: 3 bis 8.

6. Verfahren zum Diagnostizieren von Krebs nach Anspruch 3, wobei das Messen des Vorliegens oder Spiegels von BAG2 oder Cathepsin B in der Probe durch Western Blotting, Enzyme Linked Immunosorbent Assay (ELISA), Radioimmunoassay (RIA), Radioimmundiffusion, Ouchterlony-Immundiffusion, Rocket-Immunelektrophorese, immunhistochemische Färbung, ein Immunpräzipitationsassay, ein Komplementfixierungsassay, fluoreszenzaktivierte Zellsortierung (FACS), einen Proteinchip oder eine Kombination davon durchgeführt wird.

7. Verfahren zum Diagnostizieren von Krebs nach Anspruch 1, wobei der Krebs ausgewählt ist aus Brustkrebs, Kolorektalkrebs, Lungenkrebs, Sarkom, Melanom, Kopf-Hals-Krebs, Gebärmutterhalskrebs, Gebärmutterkrebs, Leberkrebs, Nierenkrebs, Bauchspeicheldrüsenkrebs und Neuroblastom.

8. Verfahren zum Diagnostizieren von Krebs nach Anspruch 7, wobei der Brustkrebs metastasierender Brustkrebs ist.

9. Verfahren zum Diagnostizieren von Krebs nach Anspruch 7, wobei der Brustkrebs ein dreifach negativer Brustkrebs ist.

## Revendications

1. Méthode de diagnostic d'un cancer, la méthode comprenant :
la mise en contact d'un échantillon isolé d'un individu avec un anticorps, un polypeptide ou une combinaison de ceux-ci, qui se lie spécifiquement à un polypeptide BAG2 ou à un fragment de celui-ci ;
la mesure de la présence ou du taux de BAG2 dans l'échantillon à partir d'un complexe formé par la mise en contact avec l'anticorps, le polypeptide ou la combinaison de ceux-ci, qui se lie spécifiquement au polypeptide BAG2 ou au fragment de celui-ci ;
ledit BAG2 comprenant i) la séquence d'acides aminés de SEQ ID n°: 1 ou 2, ou ii) un variant présentant une identité de séquence supérieure ou égale à 80 % avec la SEQ ID n°: 1 ou 2, et
la comparaison de la présence ou du taux de BAG2 mesuré à partir de l'échantillon à un taux de BAG2 mesuré à partir d'un groupe témoin,
ledit échantillon étant du sérum, du plasma ou une combinaison de ceux-ci, et
ledit BAG2 étant soluble dans le sérum, le plasma ou une combinaison de ceux-ci.

2. Méthode de diagnostic d'un cancer selon la revendication 1, une séquence d'acides aminés du polypeptide BAG2 présentant une séquence d'acides aminés de SEQ ID n° : 1 ou 2.

3. Méthode de diagnostic d'un cancer selon la revendication 1, comprenant en outre la mise en contact de l'échantillon isolé de l'individu avec un anticorps, un polypeptide ou une combinaison de ceux-ci, qui se lie spécifiquement à un polypeptide cathepsine B ou à un fragment de celui-ci ;
la mesure de la présence ou du taux de cathepsine B à partir d'un complexe formé par la mise en contact avec l'anticorps, le polypeptide ou la combinaison de ceux-ci, qui se lie spécifiquement au polypeptide cathepsine B ou au fragment de celui-ci ; et
la comparaison de la présence ou du taux de cathepsine B mesuré à partir de l'échantillon à un taux de cathepsine B mesuré à partir d'un groupe témoin.

4. Méthode de diagnostic d'un cancer selon la revendication 3, ladite cathepsine B étant une pro-cathepsine B.

5. Méthode de diagnostic d'un cancer selon la revendication 3, une séquence d'acides aminés du polypeptide cathepsine B présentant une quelconque séquence d'acides aminés choisie parmi les SEQ ID n° : 3 à 8.

6. Méthode de diagnostic d'un cancer selon la revendication 3, ladite mesure de la présence ou du taux de BAG2 ou de cathepsine B dans l'échantillon étant effectuée par transfert Western, dosage immuno-enzymatique (ELISA), dosage radio-immunologique (RIA), radioimmunodiffusion, immunodiffusion d'Ouchterlony, immuno-électrophorèse en fusée, immunohistocoloration, un test d'immunoprécipitation, un test de fixation du complément, tri cellulaire activé par fluorescence (FACS), une puce à protéines ou une combinaison de ceux-ci.

7. Méthode de diagnostic d'un cancer selon la revendication 1, ledit cancer étant choisi parmi le cancer du sein, le cancer colorectal, le cancer du poumon, un sarcome, un mélanome, le cancer de la tête et du cou, le cancer du col de l'utérus, le cancer de l'utérus, le cancer du foie, le cancer du rein, le cancer du pancréas et un neuroblastome.

8. Méthode de diagnostic d'un cancer selon la revendication 7, ledit cancer du sein étant un cancer du sein métastasique.

9. Méthode de diagnostic d'un cancer selon la revendication 7, ledit cancer du sein étant un cancer du sein triple négatif.
